# EUROPÄISCHE PATENTSCHRIFT

(11) **EP 2 178 589 B1**
(45) Veröffentlichungstag und Bekanntmachung des Hinweises auf die Patenterteilung: **26.02.2014**
(21) Anmeldenummer: 08786221.5
(22) Anmeldetag: 17.07.2008
(51) Int. Cl.: A61M 15/00

(54) **PULVERINHALATOR**
POWDER INHALER
INHALATEUR À POUDRE

(30) Priorität: 20.07.2007 DE 102007033861; 02.08.2007 DE 102007036411
(43) Veröffentlichungstag der Anmeldung: 28.04.2010
(73) Patentinhaber: Boehringer Ingelheim International GmbH, 55216 Ingelheim am Rhein (DE)
(72) Erfinder: KUHN, Rolf, 55216 Ingelheim am Rhein (DE); METZGER, Burkhard Peter, 55216 Ingelheim am Rhein (DE); KUEHN, Torsten, 55216 Ingelheim am Rhein (DE); KLADDERS, Heinrich, 55216 Ingelheim am Rhein (DE); SCHULZ, Joern-Eric, 55216 Ingelheim am Rhein (DE)
(74) Vertreter: Simon, Elke Anna Maria
(86) Internationale Anmeldenummer: PCT/EP2008/059388
(87) Internationale Veröffentlichungsnummer: WO 2009/013218

(56) Entgegenhaltungen:
- EP-A- 0 911 047
- WO-A-2004/062640
- US-A1- 2006 237 016
- US-A1- 2007 107 722

## Beschreibung

Die Erfindung betrifft einen Inhalator mit verbesserter Bedienbarkeit für die Inhalation pulverförmiger Arzneimittel aus Kapseln, welche in eine in dem Inhalator angeordnete Kapselhalterung vor der Benutzung eingesteckt werden. Nach dem Einlegen der Kapsel in die Kapselhalterung kann der Patient ein Betätigungsorgan drücken, welches aus einer Ruhestellung in eine Bewegung versetzbar ist und dabei mit mindestens einer in die Kapselhalterung einstoßbaren Nadel zusammenwirkt. Mit Hilfe der mindestens einen Nadel wird die Kapsel angestochen und das Arzneimittel freigesetzt.

Ein derartiger Inhalator wird beispielsweise in der EP 0 703 800 B 1, US 2006/0237016 A1 oder EP 0 911 047 A1 beschrieben. Der aus den vorstehend genannten Druckschriften bekannte Inhalator weist ein schalenförmiges Unterteil und einen hierzu passenden ebenfalls schalenartigen Deckel auf, welche über ein im Randbereich angeordnetes Gelenk zur Benutzung auseinander geklappt werden können. Zwischen dem Unterteil und dem Deckel greifen in dem Gelenk noch ein ebenfalls wegklappbares Mundstück und eine darunter befindliche Platte mit darunter angeordneter Kapselhalterung an. Nach dem Auseinanderklappen der einzelnen Baugruppen kann der Patient eine mit Arzneimittel gefüllte Kapsel in die Kapselhalterung einstecken, die Platte mit Kapselhalterung sowie das Mundstück in das Unterteil zurückschwenken und die Kapsel über ein seitlich aus dem Unterteil herausragendes federvorgespanntes Betätigungsorgan anstechen. Durch ein Saugen an dem Mundstück gelangt dann das Arzneimittel in die Atemwege des zu behandelnden Patienten.

Im Hinblick auf die Handhabung sollen die bekannten Inhalatoren noch weiter verbessert werden.

Die Aufgabe wird erfindungsgemäß mit einem Inhalator gelöst, bei dem das Betätigungsorgan vergrößert ist, derart aus einer zweiteiligen Konstruktion besteht, dass es einen Innenteil und einen Außenteil besitzt, und derart ausgebildet ist, dass sich der Nadelhalter oberhalb des Krafteinwirkungspunktes und unterhalb der Aufhängung des äußeren Betätigungsorgans befindet.

Hierdurch ergibt sich für den Benutzer ein tatsächlich, reduzierter Kraftaufwand, die Nadel bzw. die Nadeln durch die Kapselwandung zu drücken. Es ergibt sich außerdem ein subjektiv reduzierter Kraftaufwand für den Benutzer aufgrund der vergrößerten Oberfläche des Drückerknopfes im Vergleich zu den aus dem Stand der Technik bekannten Geräten. Für einen aus dem Stand der Technik bekannten Inhalator muss der Benutzer circa 35 Newton aufwenden, um mittels des Betätigungsorgans die Kapsel mit der Nadel bzw. den Nadeln zu durchstoßen. Beim erfindungsgemäßen Inhalator sind 10-25 Newton, vorzugsweise 15-20 Newton, notwendig.

Der Deckel besitzt einen nach innen oder außen gehenden Wulst, der äußerlich nicht erkennbar ist. Dieser Wulst dient dem Verschluss des Deckels am Drücker, welcher sich am Unterteil des Inhalators befindet.

Um die Lösung des Deckels von dem Unterteil zu ermöglichen, besitzt das Betätigungsorgan an seiner Oberseite eine Ausnehmung, die derart geneigt ist, dass sie in Form einer schiefen Ebene eine Gleitfläche für das Verschlusselement bildet und bei Betätigung und damit Vorschieben des Betätigungsorgans den Deckel vom Unterteil löst. Die Ausnehmung des Betätigungsorgans kann unterschiedlich groß sein. Die Minimalgröße muss derart sein, dass die Lösung des Deckels von dem Unterteil nach dem taschenuhrähnlichen Prinzip möglich ist. Ihre maximale Größe richtet sich nach der Oberseite des Betätigungsorgans. Die eigentliche Öffnungsbewegung des Deckels kann dann wie bisher durch Angreifen an den Deckel durch den Patienten und vollständiges Wegklappen vorgenommen werden.

Das wegklappbare Mundstück kann mit einer oder zwei Griffhilfen versehen sein, die ein schnelles und sicheres Öffnen des Mundstücks gewährleisten. Dabei kann jede Griffhilfe so angeordnet sein, dass die Berührung des Mundstücks außerhalb des Mundstückbereichs liegt, welches der zu behandelnde Patient zum Saugen in den Mund nehmen muss. Die Berührungsfläche zum Öffnen und die Berührungsfläche zum Saugen sind durch die Formgebung und durch das Erscheinungsbild des Mundstücks eindeutig voneinander getrennt. Vorzugsweise befinden sich die Griffhilfen links und rechts des Drückers, wobei die beiden Griffhilfen im Bereich des Drückers nicht zusammenlaufen. Dadurch erhält das Mundstück ein optisch und praktisch verbessertes Erscheinungsbild, welches dem Anwender eine intuitive Handhabung ermöglicht und gleichzeitig die hygienischen Verhältnisse optimiert. Dieses ist besonders im Bereich des Mundstückes wichtig, da dieses Bauteil bei Benutzung des Inhalators in den Mundraum eingeführt wird.

In einer Ausführungsform kann zur Unterstützung der Öffnungsbewegung noch mindestens ein weiteres Federelement zwischen Platte und Unterteil angeordnet sein, sodass bei einer entsprechenden Dimensionierung ein schnappartiges Aufspringen des Deckels und/oder des Mundstückes erfolgt. Alternativ ist auch eine Ausführungsform ohne Federelement möglich.

Das Betätigungsorgan ist insbesondere bei einem beginnenden Asthmaanfall von großer Wichtigkeit. Durch die wirksame Anordnung des Betätigungsorgans verbunden mit einem reduzierten Kraftaufwand für den Patienten ist die Anwendung des Inhalators deutlich erleichtert. Dies gilt insbesondere, wenn Patienten an Arthritis oder ähnlichen Erkrankungen leiden oder anderweitig in der Beweglichkeit ihrer Finger eingeschränkt sind.

Das Betätigungsorgan besteht aus einer zweiteiligen Konstruktion derart, dass es einen Innenteil und einen Außenteil besitzt, welche miteinander verschnappt sind. Das Innenteil besitzt zwei parallele Führungsarme (siehe unten). Das Außenteil drückt bei Betätigung des Betätigungsorgans über einen Kreisteil auf das Innenteil, welches sich dann linear bewegt, um die Nadeln in die Kapsel zu stechen.

Das Betätigungsorgan ist mit der mit dem Unterteil verrastbaren Platte verbunden. Dies kann beispielsweise mittels Schnapphaken, Rasthaken oder ähnlichen, technischen Lösungen geschehen.

Vorzugsweise ist das Betätigungsorgan beweglich an der Platte oder der Kapselhalterung gelagert. Die Platte und/oder die Kapselhalterung bildet bzw. bilden somit ein Widerlager für das Betätigungsorgan, welches bei einem Verschieben aus der Ruhestellung in die jeweilige Funktionsstellung an der Platte entlang gleitet und durch diese beispielsweise über eine Führungsschiene geführt ist.

In einer günstigen Ausgestaltung ist das Betätigungsorgan federvorgespannt. Aufgrund der bereits in der Ruhestellung auftretenden Rückstellkraft ist sichergestellt, dass nach der Benutzung des Betätigungsorgans dieses in die Ruhestellung zurückgeführt wird und somit der Inhalationsvorgang begonnen bzw. fortgeführt werden kann.

Vorteilhafterweise weist das Betätigungsorgan einen Hauptkörper und zwei daran angreifende parallele Führungsarme auf. Dabei ragen die Führungsarme in das Unterteil hinein und dienen mit entsprechenden Einbauteilen, beispielsweise mit außenseitig an der Kapselhalterung angeordneten Führungshülsen, der Führung des Betätigungsorgans während der Bewegung aus der Ruhestellung in die jeweiligen Funktionsstellungen und zurück in die Ruhestellung.

Die Führungsarme können an ihrem hauptkörperfemen Ende Endanschläge aufweisen, welche in der Ruhestellung an den Führungshülsen anliegen. Dadurch wird eine Federspannung des Betätigungsorgans aufgebaut.

Die Führungsarme können eine beliebige Form und Anordnung haben (z.B. zusammen- oder auseinanderlaufend). Es können weiterhin mehr als zwei Führungsarme vorhanden sein.

Insgesamt besitzt das Betätigungsorgan zwei Anschlagsbereiche; Jeweils einer ist für das Drückerinnen- und das Drückeraußenteil vorhanden.

In einer bevorzugten Ausgestaltung kann der Hauptkörper des Betätigungsorgans eine Riffelfläche aufweisen. Diese Riffelfläche kann oben oder seitlich sein. Vorzugsweise wird die Riffelfläche in einer Griffmulde des Betätigungsorgans sein.

Die Riffelfläche dient sowohl als Design-Elemente als auch der optimalen Griffigkeit bei der Betätigung. Sie liegen am Hauptkörper des Betätigungsorgans außerhalb des Inhalationsbereiches und kommen demzufolge nicht mit dem Mundbereich des Patienten in Berührung. Darüber hinaus können die Riffelflächen flächenmäßig kleiner als die Gesamtfläche des Betätigungsorgans ausgeführt sein und bieten dennoch eine Gewähr für eine sichere und schnelle Benutzung des Inhalators.

Günstigerweise ist die obere Riffelfläche in der Ruhestellung in ihrem deckelseitigen Bereich mit einer Ausnehmung zur Aufnahme des Verschlusselementes des Deckels ausgebildet. Innerhalb der Ausnehmung ist die in Richtung der seitlichen Riffelfläche gerichtete Seitenwand derart geneigt, dass sie beim Einschieben des Hauptkörpers eine Gleitfläche für das Verschlusselement bildet und dadurch das Verschlusselement zusammen mit dem Deckel aus der verrasteten Stellung gehoben wird.

Vorteilhafterweise ist die mit dem Unterteil verrastete Platte von dem Unterteil derart lösbar, dass die Platte von dem Unterteil wegschwenkbar ist. Diese Schwenkfunktion erleichtert die Reinigung des Inhalators. Die Verrastung zwischen Platte und Unterteil kann mittels vorstehender Haltelaschen realisiert werden.

Auch ist es möglich, den Inhalator gemäß allen Ausführungsformen derart auszuführen, dass das Betätigungsorgan mit der mindestens einen, in die Kapselhalterung einstoßbaren Nadel so mit der Platte verbunden ist, dass es zusammen mit der mit dem Unterteil verrasteten Platte vom Unterteil gelöst und weggeschwenkt werden kann. Vorzugsweise ist das Betätigungsorgan derart mit der Platte verbunden, dass beide Teile zusammen eine schwenkbare Einheit bilden.

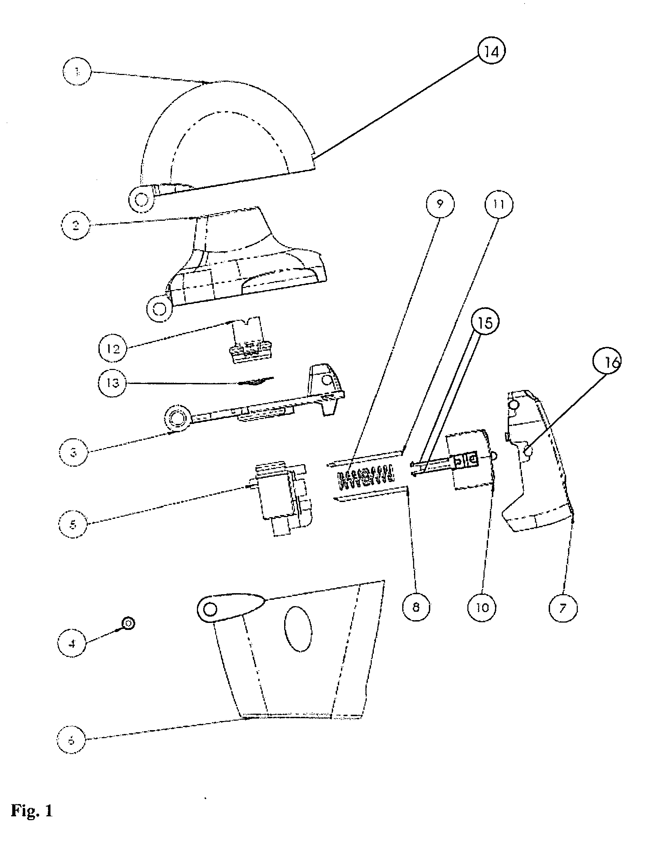

Zum besseren Verständnis der Erfindung wird diese nunmehr anhand der nachfolgenden Zeichnung (Fig. 1) näher erläutert. Dabei zeigt die:
- **Fig. 1:**: eine Explosionsdarstellung mit Betätigungsorgan und Mundstück mit Griffhilfe

In der Fig. 1 ist der Inhalator in einer Explosionsdarstellung gezeigt. Die wesentlichen Baugruppen sind dabei das Unterteil 6, welches die Platte 3 aufnimmt und durch diese abgedeckt wird, das an dem Unterteil 6 über die Haltenasen des Siebgehäuses 12 verrastbare Mundstück 2 und den komplementär zu dem Unterteil 6 ausgebildeten Deckel 1.

In geschlossenem Zustand des Inhalators greift das Verschlusselement 14 am Deckel 1 an die Platte 3 und wird dort kraftschlüssig gehalten. Auch ist es möglich, durch wulstartige Ausbildungen am Verschlusselement 14 einen Formschluss zu realisieren. Für ein Angreifen des Verschlusselementes 14 am Deckel 1 an die Platte 3 weist das äußere Betätigungsorgan 7 eine Ausnehmung auf, in welche das Verschlusselement 14 beim Verschließen hineinversenkt wird. Die Ausnehmung ist mit einer geneigten Seitenwand versehen und befindet sich in dem deckelseitigen Bereich.

Das Betätigungsorgan besteht aus einem äußeren Betätigungsorgan 7 und einem inneren Betätigungsorgan 10. Zum Öffnen des Deckels 1 wird zunächst das äußere Betätigungsorgan 7 in Richtung des Inhalators bewegt bzw. gedrückt. Dabei stößt das Verschlusselement 14 am Deckel 1 auf die geneigte Seitenwand der Ausnehmung, welche bei weiterem Vorschub des Verschlusselementes 14 als Gleitfläche wirkt und für ein Lösen des Deckels 1 sorgt.

Die Aussparung 16 verbindet das äußere und das innere Betätigungsorgan 7, 10 mittels einer Aufhängung, die beispielsweise als Schnapphaken, Stift oder einer anderen Aufhängevorrichtung ausgebildet ist. Die Aussparung 16 kann von runder oder ovaler Form sein. Das Oval kann in horizontaler oder vertikaler Position bzw. jeder Position angeordnet sein.
Vorzugsweise handelt es sich bei der Aussparung 16 um ein sog. Langloch, d. h. ein längliches Oval, welches die Nadelführung in axialer Richtung optimal ermöglicht, um so ein präzises Anstechen der Kapsel zu gewährleisten.

Das Unterteil 6 ist becherartig ausgeformt und nimmt vollständig die an der Unterseite der Platte 3 angeordnete Kapselhalterung 5 auf. Um eine mit Arzneimittel gefüllte Kapsel (nicht dargestellt) in die Kapselhalterung 5 einlegen zu können, muss jedoch auch noch das Mundstück 2 weggeklappt werden. In der Ausführungsform gemäß der Fig. 1 erfolgt dieses durch Angreifen an das äußere Betätigungsorgan 7.

In dieser geöffneten Position von Deckel 1 und Mundstück 2 kann die Kapsel durch eine Öffnung in der Platte 3 in die Kapselhalterung 5 eingelegt werden. Nachfolgend wird das Mundstück 2 wieder zurückgeschwenkt und durch Verrastung der Haltenasen des Siebgehäuses 12 in Platte 3 wieder verschlossen. Das Siebgehäuse 12 beinhaltet mittig das Siebgeflecht 13. Das Siebgeflecht 13 besteht aus handelsüblichen Materialien, wie z. B. Metall oder Kunststoff. Im letzteren Fall kann das Sieb mittels des Spritzgußverfahrens hergestellt werden. Zum Freisetzen des Wirkstoffes wird das äußere Betätigungsorgan 7 betätigt. Seine Ausbildung ist derart, dass sich der Nadelhalter oberhalb des Krafteinwirkungspunktes und unterhalb der Aufhängung des äußeren Betätigungsorgans befindet. An dem inneren Betätigungsorgan 10 befindet sich mindestens eine Nadel, vorzugsweise sind es jedoch zwei, senkrecht versetzte und parallel verlaufende Nadeln 8, 11, die sich kontinuierlich mit dem Einschieben des Betätigungsorgans 7, 10 in Richtung der Kapsel (nicht dargestellt) bewegt bzw. bewegen und diese perforiert bzw. perforieren. Der Perforiervorgang kann durch ein Sichtfenster (nicht dargestellt) beobachtet werden.

In der Kapselhalterung 5 ist eine bzw. sind mindestens zwei rohrförmige Nadeldurchführungen zu erkennen, die axial entsprechend der Bewegungsrichtung der Nadel bzw. Nadeln 8, 11 ausgerichtet ist bzw. sind. Somit ist einerseits für ein treffsicheres Aufsetzen der Nadel bzw. Nadeln 8, 11 auf der Kapsel (nicht dargestellt) gesorgt und andererseits für eine zusätzliche Führung des Betätigungsorgans 7, 10. Die wesentliche Führung wird jedoch über zwei seitlich angeordnete Führungsarme 15 realisiert. Die Führungsarme 15 haben darüber hinaus die Aufgabe, das Betätigungsorgan 7, 10 unter einer Vorspannung zu halten. Hierfür sind die Führungsarme 15 an ihrem hauptkörperfernen Ende mit Endanschlägen versehen, die in der Ruhestellung des Betätigungsorgans 7, 10 an den Führungshülsen der Kapselhalterung 5 anliegen. Die Führungshülsen befinden sich an der Außenseite der Kapselhalterung 5. Zwischen den Führungsarmen 15 ist eine Schraubenfeder 9 angeordnet, die in ihrer axialen Erstreckung parallel zu der Nadel bzw. den Nadeln 8, 11 verläuft, wobei die Schraubenfeder 9 derart mit der Länge der Führungsarme 15 abgestimmt ist, dass das Betätigungsorgan 7, 10 auch in der Ruhestellung noch vorgespannt ist.

Die einzelnen Baugruppen Unterteil 6, Platte 3, Mundstück 2 und Deckel 1 werden über Gelenkaufnahmen und einer Achse 4 miteinander verbunden und sind alle um diese Achse herum gegenseitig verschieb- bzw. schwenkbar.

Als Nadeln können alle dem Fachmann bekannten Nadeln verwendet werden. Es kann sich hierbei um Voll- oder Hohlnadeln handeln. Vorzugsweise werden Vallnadeln verwendet. Insbesondere kann als obere Nadel (dem Mundstück zugewandt) eine Dreikantnadel mit einem Dreikantschliff verwendet werden. Als untere Nadel kann eine Standardnadel mit einem Standardschliff verwendet werden, wie zum Beispiel in der deutschen DIN-Norm festgelegt.
Alternativ kann die obere Nadel eine Standardnadel mit einem Standardschliff und die untere Nadel eine Dreikantnadel mit einem Dreikantschliff sein.
Als zweite Alternative ist die Verwendung von zwei Dreikantnadeln mit einem Dreikantschliff oder zwei Standardnadeln mit einem Standardschliff möglich.

Als Kapseln können alle dem Fachmann bekannten Kapseln für Pulverinhalatoren verwendet werden (z.B.(Hart-)Gelatine-, Kunststoff-, Metallkapseln).
Insbesondere kann in dem erfindungsgemäßen Inhalator eine Kunststoffkapsel zur Anwendung kommen, wie in WO 00/07572, EP 1 100 474 offenbart.

Der Inhalator kann ein Sichtfenster aufweisen. Dieses ist jedoch nicht erforderlich für seine bestimmungsgemäße Funktion.

Ebenso können alle Bauteile des Inhalators gemäß dem Fachmann bekannten Verfahren und kunststofftechnischen Möglichkeiten modifiziert werden. Mögliche Modifikationen sind beispielsweise Versteifungen oder Veränderungen der Wanddicke. Diese Möglichkeiten sind für die Funktionsweise des Inhalators jedoch nicht zwingend erforderlich.

Der Inhalator kann außerdem nach dem Fachmann bekannten Verfahren auf seiner Innen- und/oder Außenseite beschichtet werden.

Für die Inhalation kommen alle Arten von pulverförmigen Arzneimitteln in Betracht, deren Applikation auf inhalativem Wege therapeutisch sinnvoll ist.

Die unten genannten Verbindungen können allein oder in Kombination zur Anwendung in der erfindungsgemäßen Vorrichtung gelangen. In den unten genannten Verbindungen ist

W einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Anti-histaminika, PAF-Antagonisten und PI3-Kinase Inhibitoren. Weiterhin können zwei- oder dreifach Kombinationen von W kombiniert werden und zur Anwendung in der erfindungsgemäßen Vorrichtung gelangen. Beispielhaft genannte Kombinationen von W wären:
- W stellt ein Betamimetika dar, kombiniert mit einem Anticholinergika, Corticosteroide, PDE4-Inhibitore, EGFR-Hemmern oder LTD4-Antagonisten,
- W stellt ein Anticholinergika dar, kombiniert mit einem Betamimetika, Corticosteroiden, PDE4-Inhibitoren, EGFR-Hemmern oder LTD4-Antagonisten,
- W stellt ein Corticosteroiden dar, kombiniert mit einem PDE4-Inhibitoren, EGFR-Hemmem oder LTD4-Antagonisten
- W stellt ein PDE4-Inhibitoren dar, kombiniert mit einem EGFR-Hemmern oder LTD4-Antagonisten
- W stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert-butylamino)ethanol
- 6-Hydroxy-8-(1-hydroxy-2-[2-(4-methoxy-phenyl)-1,1-dimethyl-ethylamino]-ethyl}-4H-benzo[1,4]oxazin-3-on
- 6-Hydroxy-8- {1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl} -4H-benzo[1,4]oxazin-3-on
- 8-{2-[1,1-Dimethyl-2-(2,4,6-trimethylphenyl)-ethylamino]-1-hydrox*y-ethyl }-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl]-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-{4-Ethoxy-carbonylamino-3-cyano-5-fluorophenyl)-2-(tert.-butylamino)ethanol
- 2-Hydroxy-5-{1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[3-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel AC-1 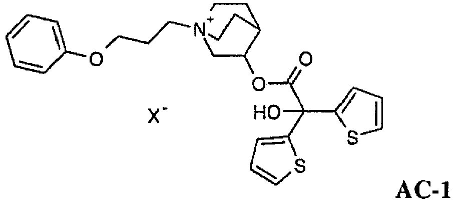 worin X - ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel AC-1-en 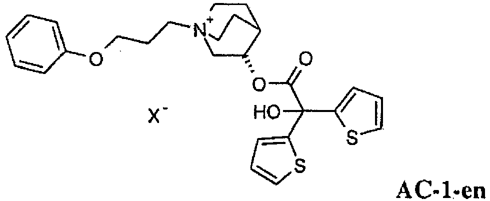 enthalten, worin X- die vorstehend genannten Bedeutungen aufweisen kann. Weiterhin bevorzugte Anticholinergika sind ausgewählt aus den Salzen der Formel AC-2 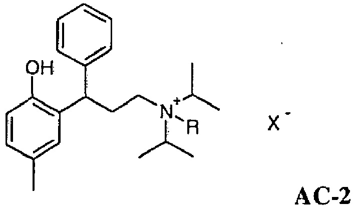 worin R entweder Methyl oder Ethyl bedeuten und worin X - die vorstehend genannte Bedeutungen aufweisen kann. In einer alternativen Ausführungsform kann die Verbindung der Formel AC-2 auch in Form der freien Base AC-2-base vorliegen.

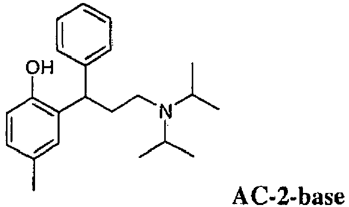

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclo-propylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrolidon
- 3-(Cyclopentyloxy-4-methoxyphenyl)-1-(4-N'-[N-2-cyano-S-methyl-isathioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der PDE4-Inhibitoren ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium-oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]-amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-{1-Phenyl-ethyl)amino]-6-{{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-Methyl-amino)-1-oxo-2-buten-1-yl}amino)-7-cyclopropylmethoxy-chinazolin
- 4-[(3-Chlor-4-fluorphenyl)amino]-6-[4-{N,N-dimethylarnino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-(N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinyl-carbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-{3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran)-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yl-oxy}-7-methoxy-chinazolin
- 4-[(3-Chlor-4-fluor-phenyl)amino]-6-{1-[(methoxymethyl)carbonyl]-piperidin-4-yl-oxy)-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-pheny)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-niethoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amin]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxyl-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chimazolin
- 4-[(3-Chlor-4-fluor-phenyl)amino]-6-[2-(2,2-dimethy)-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind diese Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als pharmazeutisch wirksame Substanzen, Substanzformulierungen oder Substanzmischungen werden alle inhalierbaren Verbindungen eingesetzt, wie z.B. auch inhalierbare Makromoleküle, wie in EP 1 003 478 offenbart. Vorzugsweise werden Substanzen, Substanzformulierungen oder Substanzmischungen zur Behandlung von Atemwegserkrankungen eingesetzt, die im inhalativen Bereich Verwendung finden.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkomalkaloide: Dihydroergotamin, Ergotamin.

### Bezugszeichenliste

- 1: Deckel
- 2: Mundstück
- 3: Platte
- 4: Achse
- 5: Kapselhalterung
- 6: Unterteil
- 7: äußeres Betätigungsorgan
- 8: Nadel
- 9: Schraubenfeder
- 10: inneres Betätigungsorgan
- 11: Nadel
- 12: Siebgehäuse
- 13: Siebgeflecht
- 14: Verschlusselement
- 15: Führungsarm(e)
- 16: Aussparung

## Patentansprüche

1. Inhalator für die Inhalation pulverförmiger Arzneimittel aus Kapseln, aufweisend
- ein Unterteil (6),
- ein mit dem Unterteil (6) verrastbare Platte (3), mit welcher das Unterteil (6) verschließbar ist, und eine in dem Unterteil (6) versenkbare Kapselhalterung (5) zur Aufnahme der Kapseln,
- ein mit der Platte (3) verrastbares Mundstück (2) und
- ein Betätigungsorgan, welches aus einer Ruhestellung in eine Bewegung versetzbar ist und dabei mit mindestens einer in die Kapselhalterung (5) einstoßbaren Nadel (8, 11), welche sich in einer Nadelhalterung befindet, zusammenwirkt,
**dadurch gekennzeichnet, dass**
- das Betätigungsorgan zweiteilig ist, wobei es aus einem äußeren Betätigungsorgan (7) und einem inneren Betätigungsorgan (10) besteht, wobei das äußere Betätigungsorgan (7) größer als das innere Betätigungsorgan (10) ist und
- das äußere Betätigungsorgan (7) an der Platte (3) an einer Aufhängung aufgehängt ist,
- sich die mindestens eine Nadel (8, 11) an dem inneren Betätigungsorgan (10) befindet, und
- das äußere Betätigungsorgan (7) bei Betätigung über den Teil eines Kreises auf das innere Betätigungsorgan (10) drückt, welches sich dadurch linear bewegt, um die mindestens eine Nadel (8, 11) in eine in die Kapselhalterung (5) eingesetzte Kapsel zu stechen.

2. Inhalator nach Anspruch 1, **dadurch gekennzeichnet, dass** zur Perforation einer Kapsel in der Kapselhalterung (5) durch Bewegung des äußeren Betätigungsorgans (7) 10-25 Newton, vorzugsweise 15-20 Newton, aufzuwenden sind.

3. Inhalator nach Anspruch 1 oder 2, **dadurch gekennzeichnet, dass** das Betätigungsorgan verschiebbar an der Platte (3) und/oder der Kapselhalterung (5) gelagert ist.

4. Inhalator nach Anspruch 1, 2, oder 3, **dadurch gekennzeichnet, dass** das Betätigungsorgan (7, 10) federvorgespannt ist.

5. Inhalator nach Anspruch 1, **dadurch gekennzeichnet, dass** das innere Betätigungsorgan (10) am äußeren Betätigungsorgan (7) befestigt ist und das innere Betätigungsorgan Führungsarme (15) und die Nadelhalterungen enthält.

6. Inhalator nach Anspruch 5, **dadurch gekennzeichnet, dass** das äußere Betätigungsorgans (7) geriffelt ist und mindestens eine seitliche Riffelfläche aufweist.

7. Inhalator nach Anspruch 1, **dadurch gekennzeichnet, dass** der Inhalator einen Deckel (1) aufweist, welcher das Mundstück (2) in einer Verschlussposition abdeckt und mittels eines Verschlusselementes (14) verrastet, und das äußere Betätigungsorgan (7) an seiner Oberseite eine Ausnehmung besitzt, die derart geneigt ist, dass sie in Form einer schiefen Ebene eine Gleitfläche für das Verschlusselement (14) bildet.

8. Inhalator nach Anspruch 1, **dadurch gekennzeichnet, dass** das Anstechen einer Kapsel in der Kapselhalterung (5) durch zwei senkrecht versetzte und parallel verlaufende Nadeln (8, 11) erfolgt, die durch die Betätigung des äußeren Betätigungsorgans (7) bewegt werden und die Kapsel perforieren.

9. Inhalator nach Anspruch 8, **dadurch gekennzeichnet, dass** die Nadeln (8, 11) durch rohrförmige Nadeldurchführungen geführt werden.

10. Inhalator nach Anspruch 9, **dadurch gekennzeichnet, dass** die Nadeln (8, 11) durch jeweils einen seitlich angeordneten Führungsarm (15) geführt werden.

11. Inhalator nach Anspruch 10, **dadurch gekennzeichnet, dass** der bzw. die seitlich angeordneten Führungsarme (15) das Betätigungsorgan (7, 10) unter Vorspannung halten.

12. Inhalator nach einem oder mehreren der Ansprüche 1 - 11, **dadurch gekennzeichnet, dass** die Kapselhalterung (5) des Inhalators eine Kapsel enthält.

## Claims

1. Inhaler for inhaling powdered medicaments from capsules, comprising
- a lower part (6),
- a plate (3) that can be latched to the lower part (6), by means of which the lower part (6) can be closed off, and a capsule holder (5) that can be lowered within the lower part (6) for holding capsules,
- a mouthpiece (2) that can be latched to the plate (3), and
- an actuating member which can be set in motion from a resting position and thereby interacts with at least one pin (8, 11) that is located in a pin holder and can be pushed into the capsule holder (5),
**characterised in that**
- the actuating member is in two parts, consisting of an outer actuating member (7) and an inner actuating member (10), the outer actuating member (7) being larger than the inner actuating member (10), and
- the outer actuating member (7) is suspended from the plate (3) at a suspension point,
- the at least one pin (8, 11) is located on the inner actuating member (10), and
- on actuation over part of a circle the outer actuating member (7) presses onto the inner actuating member (10), as a result of which the inner actuating member (10) moves in a linear movement in order to push the at least one pin (8, 11) into a capsule inserted in the capsule holder (5).

2. Inhaler according to claim 1, **characterised in that** 10-25 Newtons, preferably 15-20 Newtons, have to be applied in order to perforate a capsule in the capsule holder (5) by moving the outer actuating member (7).

3. Inhaler according to Claim 1 or 2, **characterised in that** the actuating member is displaceably mounted on the plate (3) and/or on the capsule holder (5).

4. Inhaler according to Claim 1, 2 or 3, **characterised in that** the actuating member (7, 10) is spring-loaded.

5. Inhaler according to Claim 1, **characterised in that** the inner actuating member (10) is attached to the outer actuating member (7) and the inner actuating member contains guide arms (15) and the pin holders.

6. Inhaler according to Claim 5, **characterised in that** the outer actuating member (7) is grooved and comprises at least one lateral grooved surface.

7. Inhaler according to Claim 1, **characterised in that** the inhaler comprises a cover (1) which covers the mouthpiece (2) in a closure position and latches by means of a closure element (14), and the outer actuating member (7) has, on its upper side, a recess which is inclined so as to form a sliding surface for the closure element (14) in the form of a sloping plane.

8. Inhaler according to Claim 1, **characterised in that** the piercing of a capsule in the capsule holder (5) is performed by two perpendicularly offset, parallel pins (8, 11) that are moved by the actuation of the outer actuating member (7) and perforate the capsule.

9. Inhaler according to Claim 8, **characterised in that** the pins (8, 11) are passed through tubular pin passages.

10. Inhaler according to Claim 9, **characterised in that** the pins (8, 11) are each passed through a laterally mounted guide arm (15).

11. Inhaler according to Claim 10, **characterised in that** the laterally mounted guide arm or arms (15) hold the actuating member (7, 10) under tension.

12. Inhaler according to one or more of Claims 1 - 11, **characterised in that** the capsule holder (5) of the inhaler contains a capsule.

## Revendications

1. Inhalateur pour l'inhalation de médicament sous forme pulvérulente à partir de capsules, comprenant
- une partie inférieure (6),
- une plaque pouvant s'encliqueter (3) avec la partie inférieure (6), avec laquelle la partie inférieure (6) peut être fermée, et un support de capsule escamotable (5) dans la partie inférieure (6) pour la réception de la capsule,
- un embout buccal (2) pouvant s'encliqueter avec la plaque (3) et
- un organe d'actionnement qui peut être mis en mouvement à partir d'une position de repos et coopère ainsi avec au moins une aiguille (8, 11) pouvant être introduite dans le support de capsule (5), qui se trouve dans un support d'aiguille,
**caractérisé en ce que**
- l'organe d'actionnement est en deux parties, constitué d'un organe d'actionnement externe (7) et d'un organe d'actionnement interne (10), l'organe d'actionnement externe (7) étant plus grand que l'organe d'actionnement interne (10) et
- l'organe d'actionnement externe (7) est monté sur la plaque (3) à une suspension,
- l'au moins une aiguille (8, 11) se trouve sur l'organe d'actionnement interne (10), et
- l'organe d'actionnement externe (7) appuie lors de l'actionnement sur la partie d'une cercle sur l'organe d'actionnement interne (10) qui se déplace ainsi de façon linéaire autour de l'au moins une aiguille (8, 11) dans une capsule à perforer, insérée dans le support de capsule (5).

2. Inhalateur selon la revendication 1, **caractérisé en ce que**, pour la perforation d'une capsule dans le support de capsule (5) par un mouvement de l'organe d'actionnement externe (7), il faut exercer une force de 10 à 25 Newton, de préférence 15 à 20 Newton.

3. Inhalateur selon la revendication 1 ou 2, **caractérisé en ce que** l'organe d'actionnement est monté de façon à pouvoir coulisser sur la plaque (3) et/ou le support de capsule (5)

4. Inhalateur selon la revendication 1, 2 ou 3, **caractérisé en ce que** l'organe d'actionnement (7, 10) est à ressort.

5. Inhalateur selon la revendication 1, **caractérisé en ce que** l'organe d'actionnement interne (10) est fixé à l'organe d'actionnement externe (7) et l'organe d'actionnement interne contient des bras de guidage (15) et les supports d'aiguilles.

6. Inhalateur selon la revendication 5, **caractérisé en ce que** l'organe d'actionnement externe (7) est rainuré et présente au moins une surface de rainure latérale.

7. Inhalateur selon la revendication 1, **caractérisé en ce que** l'inhalateur présente un couvercle (1) qui recouvre l'embout buccal (2) dans une position de fermeture et s'encliquette au moyen d'un élément de fermeture (14), et l'organe d'actionnement externe (7) possède sur sa partie supérieure, un évidement qui est incliné de telle sorte qu'il forme, sous la forme d'un plan oblique, une surface de glissement pour l'élément de fermeture (14).

8. Inhalateur selon la revendication 1, **caractérisé en ce que** la perforation d'une capsule dans le support de capsule (5) s'effectue par deux aiguilles (8, 11) décalées verticalement et évoluant en parallèle, qui sont déplacées par l'actionnement de l'organe d'actionnement externe (7) et perforent la capsule.

9. Inhalateur selon la revendication 8, **caractérisé en ce que** les aiguilles (8, 11) sont guidées par des passages d'aiguille tubulaires.

10. Inhalateur selon la revendication 9, **caractérisé en ce que** les aiguilles (8, 11) sont guidées respectivement par un bras de guidage (15) disposé latéralement.

11. Inhalateur selon la revendication 10, **caractérisé en ce que** le, plus précisément les, bras de guidage (15) disposé(s) latéralement maintiennent l'organe d'actionnement (7, 10) sous précontrainte.

12. Inhalateur selon l'une ou plusieurs des revendications 1 à 11, **caractérisé en ce que** le support de capsule (5) de l'inhalateur contient une capsule.
